(19) Europäisches Patentamt
European Patent Office
Office européen des brevets

(11) **EP 1 436 244 B1**

(12) **EUROPÄISCHE PATENTSCHRIFT**

(45) Veröffentlichungstag und Bekanntmachung des Hinweises auf die Patenterteilung:
**28.05.2008 Patentblatt 2008/22**

(21) Anmeldenummer: **02777265.6**

(22) Anmeldetag: **30.09.2002**

(51) Int Cl.:
***C07C 51/25*** *(2006.01)*

(86) Internationale Anmeldenummer:
**PCT/EP2002/010952**

(87) Internationale Veröffentlichungsnummer:
**WO 2003/029177 (10.04.2003 Gazette 2003/15)**

(54) **VERFAHREN ZUR HERSTELLUNG VON ACRYLSÄURE DURCH HETEROGEN KATALYSIERTE GASPHASENOXIDATION**

METHOD FOR THE PRODUCTION OF ACRYLIC ACID BY HETEROGENEOUSLY-CATALYSED GAS-PHASE OXIDATION

PROCEDE DE PREPARATION D'ACIDE ACRYLIQUE PAR OXYDATION EN PHASE GAZEUSE A CATALYSE HETEROGENE

(84) Benannte Vertragsstaaten:
**AT BE BG CH CY CZ DE DK EE ES FI FR GB GR IE IT LI LU MC NL PT SE SK TR**

(30) Priorität: **01.10.2001 DE 10148566**

(43) Veröffentlichungstag der Anmeldung:
**14.07.2004 Patentblatt 2004/29**

(73) Patentinhaber: **BASF SE**
**67056 Ludwigshafen (DE)**

(72) Erfinder:
• **NESTLER, Gerhard**
**1070 Wien (AT)**
• **SCHRÖDER, Jürgen**
**67071 Ludwigshafen (DE)**

(56) Entgegenhaltungen:
**EP-A- 0 575 897        EP-A- 0 911 313**
**DE-A- 2 311 883        DE-A- 19 955 176**

**Beschreibung**

Die Erfindung betrifft ein Verfahren zur Herstellung von Acrylsäure aus Propen mittels Gasphasenoxidation, bei dem man ein Propen und Wasserdampf enthaltendes Ausgangsgasgemisch, dessen molares Propen/Wasserdampf-verhältnis $\geq 1$ ist und dessen Propangehalt $\leq 5$ % ist, bei erhöhter Temperatur durch ein Katalysatorfestbett mit aufeinanderfolgenden Katalysatorteilbetten A und B führt, wobei das zuerst durchlaufene Teilbett A als Aktivmasse ein die Elemente Mo, Bi und Fe enthaltendes Mischoxid und das Teilbett B als Aktivmasse ein die Elemente Mo und V enthaltendes Mischoxid enthält.

Acrylsäure und die aus ihr hergestellten Ester sind wichtige Ausgangsstoffe bei der Herstellung von Polymerisaten, die beispielsweise als Superabsorber, Klebstoffe, Lackharze oder Anstrichdispersionen Anwendung finden.

Die nach einer Gasphasenoxidation isolierte Acrylsäure ist jedoch mit Carbonsäure wie Essigsäure, Propionsäure und Diacrylsäure, Allylacrylat, Carbonylverbindungen und Polymerisationsinhibitoren verunreinigt. Da diese Nebenprodukte zumeist unerwünscht sind, müssen sie in aufwendigen Reinigungsoperationen, in der Regel destillativ abgetrennt werden.

Aufgrund der Ähnlichkeit von Acrylsäure mit den bei der Gasphasenoxidation gebildeten Nebenprodukten hinsichtlich der chemischen und physikalischen Eigenschaften wie Siedepunkte und Molekulargewicht, ist eine Abtrennung der Nebenprodukte, insbesondere den Carbonsäuren sowie von Allylacrylat fast nicht möglich. Die für eine fraktionierte Destillation benötigte Trennstufenzahl bringt eine längere thermische Belastung der Acrylsäure mit sich und als Folge davon Polymerisatbildung an Kolonnenböden, Leitungen, Pumpen und Ventilen. Insbesondere die destillative Abtrennung der Propionsäure von der Acrylsäure ist im industriellen Maßstab überhaupt nicht möglich. Gleiches gilt für die Abtrennung der Propionsäureester von den entsprechenden Acrylsäureestern.

Polymerisiert man die Acrylsäure oder ihre Ester, so sind die Carbonsäuren niedermolekulare Verunreinigungen, die, würde man die Dispersionen nicht aufwendig desodorieren, langsam aus den Polymerisaten dünsten würden.

Nicht minder unerwünscht ist Allylacrylat als Nebenprodukt, da es auf Acrylsäure destabilisierend wirkt und insbesondere bei der Polymerisation als Vernetzer die Polymerisateigenschaft entscheidend beeinflußt. Aufwendige Reinigungsoperationen sind die Folge.

Angesichts dieser Problematik wurden verschiedene Ansätze gemacht, durch Modifizierung des Katalysators den Propionsäuregehalt zu senken.

Die JP-A-2000-53611 schlägt eine thermische Nachbehandlung des acrylsäurehaltigen Oxidationsgasgemisches bei 300 - 500°C in Gegenwart von Oxiden der Elemente Mo, Fe, Co und/oder Ni vor. Diese Nachbehandlung senkt zwar den Propionsäuregehalt, führt jedoch zu Acrylsäureverlusten von 8 %.

Die DE-A-23 11 883 postuliert, dass in der 1. Oxidationsstufe nicht umgesetztes Propen in der 2. Stufe zu Propionsäure oxidiert wird. Gemäß dieser Schrift kann die Propionsäuremenge bei einem Mo/V-Mischoxidkatalysator mit geringem Alkalimetallzusatz für die 2. Oxidationsstufe auf 550 ppm gesenkt werden.

Gemäß JP-A 2000-169420 wird Propionsäure auch ausgehend von Acrolein und Acrylsäure gebildet. Der Propionsäuregehalt wird gemäß dieser Lehre durch Wahl eines Mo/V/Sb/Pb-Mischoxidkatalysators in der 2. Oxidationsstufe gesenkt.

Die Lehre dieser Schriften gilt für Ausgangsgasgemische mit einem deutlich höheren Wasserdampf- als Propengehalt. Ein hoher Wasserdampfgehalt wirkt sich positiv auf die Acrylsäureselektivität der Oxidationsreaktion aus, wie der US-A-4 147 885 zu entnehmen ist.

Ganz allgemein ist dem Stand der Technik zu entnehmen, dass es wichtig ist, mit Wasserdampf oder anderen Inertgasen das Propen zu verdünnen.

Die US-A-4 031 135 beschreibt die Rückführung der nicht kondensierbaren Gase am Ende der Oxidation zum Ausgangsgasgemisch, was erhöht Acrylsäureselektivität bewirkt. Der Propenumsatz beträgt jedoch maximal 95 mol-%. Ferner wird über die Nebenprodukte keine Aussage gemacht.

Gemäß der EP-A-0 253 409 ist es vorteilhaft, den Wasserdampf durch nichtwässrige Verdünnungsgase wie Propan zu ersetzen. Dabei ist eine Wärmekapazität der rückgeführten Gase von $\geq 6,5$ cal/g mol (°C) von Bedeutung. Gemäß dieser Schrift wird der Acetaldehyd- und/oder Essigsäuregehalt gesenkt. Der Verwendung von Propan als Verdünnungsgas hat jedoch unweigerlich erhöhte Propionsäuregehalt als Nebenprodukte zur Folge. Niedrige Wasserdampfgehalte ergeben nur in Verbindung mit Propenumsätzen <96 mol-% günstige Essigsäurewerte. Der Einfluß der Reaktionsbedingungen auf die Propionsäure ist all diesen Schriften nicht zu entnehmen.

Aufgabe der vorliegenden Erfindung ist die Bereitstellung eines Verfahrens zur Herstellung von Acrylsäure durch katalytische Gasphasenoxidation von Propen, das eine Acrylsäure mit einem sehr geringen Allylacrylat- und Propionsäure-Gehalt liefert. Insbesondere soll eine Acrylsäure erhalten werden, die ohne zusätzliche Nachbehandlungsschritte weniger als 400 ppm Propionsäure und weniger als 100 ppm Allylacrylat enthält.

Demgemäß wurde ein Verfahren zur Herstellung von Acrylsäure aus Propen mittels Gasphasenoxidation gefunden, bei dem man ein Propen und Wasserdampf enthaltendes Ausgangsgasgemisch, dessen molares Propen/Wasserdampfverhältnis $\geq 1$ ist und dessen Propangehalt $\leq 5$ % ist, bei erhöhter Temperatur durch ein Katalysatorfestbett mit aufeinanderfolgenden Katalysatorteilbetten A und B, wobei das zuerst durchlaufende Teilbett A als Aktivmasse ein die Elemente Mo, Bi und Fe enthaltendes Mischoxid und das Teilbett B als Aktivmasse ein die Elemente Mo und V enthaltendes Mischoxid enthält, derart führt, dass bei einfachem Durchlauf durch Teilbett A mindestens 96,5 mol-% Propen umgesetzt werden.

Im Unterschied dazu lehrt die DE-A 19955176 im Regelfall lediglich einen Propenumsatz von wenigstens 92 mol-% und gegebenenfalls wenigstens 94 mol-%. Die EP-A 911313 fordert ein Propen/Wasserdampfverhältnis von < 0,5.

Bei der Gasphasenoxidation wird das Propen und Wasserdampf enthaltende Ausgangsgasgemisch bei erhöhter Temperatur (üblicherweise ca. 200 bis 400°C) sowie gegebenenfalls erhöhtem Druck über übergangsmetallische Mischoxidaktivmassen geleitet und oxidativ in zwei Schritten über Acrolein in Acrylsäure umgewandelt (vgl. z.B. DE-A-19 62 431, DE-A-29 43 707, DE-A-12 05 502, EP-A-0 257 565, EP-A-0 253 409, DE-A-22 51 364, EP-A-0 117 146, GB-B-1 450 986 und EP-A-0 293 224. Als Oxidationsmittel wird Sauerstoff verwendet.

Das Ausgangsgasgemisch enthält neben Propen, Sauerstoff und Wasserdampf in der Regel Inertgase wie molekularen Stickstoff, CO, $CO_2$ und inerte Kohlenwasserstoffe. Wird als inertes Verdünnungsgas $N_2$ gewählt, erweist sich die Verwendung von Luft als besonders vorteilhaft. Das molare Propen/Wasserdampfverhältnis ist im Ausgangsgasgemisch ≥ 1, bevorzugt ≥ 1,5, insbesondere ≥ 2. In einer bevorzugten Verfahrensvariante wird ein Gasgemisch zugesetzt, welches nach Abtrennung der Acrylsäure mittels Extraktion oder partieller Kondensation-aus dem Produktgemisch der Gasphasenoxidation erhalten wird. Als Propen kann technisches Propen (chemical grade, ASTM D 5273-92) eingesetzt werden, das bis zu 8 % mit Propan verunreinigt ist. Eine typische Zusammensetzung technischen Propens enthält mindestens 95 % Propen und als Verunreinigungen 3-5 % Propan, ≤0,2 % C1/C2-Kohlenwasserstoff, ≤0,1 % C4-Kohlenwasserstoff, ≤10 ppm C6-Kohlenwasserstoff, ≤0,01 % C3/C4-Diene, ≤0,01 % Ethen, ≤0,005 % Wasser, ≤1 ppm Gesamtschwefel und ≤1 ppm Gesamtchlor. Der Propangehalt des Ausgangsgasgemisches der Gasphasenoxidation darf 5 % nicht überschreiten. Bevorzugt ist der Propangehalt ≤ 3 % insbesondere ≤ 2 %, ganz besonders bevorzugt ≤1 %.

Die Mischoxidaktivmassen sind zu Formkörpern der unterschiedlichsten Geometrie geformt, die zu einem Festbett geschüttet werden, durch das das Propen enthaltende Ausgangsgasgemisch bei erhöhter Temperatur geleitet wird. Während des Kontaktes mit der Mischoxidaktivmasse kommt es zur gewünschten Oxidation. Bei Bedarf können die Katälysatorformkörper auch mit inerten Formkörpern verdünnt sein.

Das Katalysatorfestbett setzt sich aus aufeinanderfolgenden Katalysatorteilbetten A und B zusammen, wobei am Mischoxid des zuerst durchlaufenen Teilbetts A die Oxidation zum Acrolein und am Mischoxid des Teilbetts B die Oxidation zur Acrylsäure stattfindet.

Bei den Katalysatoren unterscheidet man aufgrund ihrer Herstellung verschiedene Typen. Erfolgt sie durch Verdichten von pulverförmigen Mischoxidmassen mit gegebenenfalls Hilfsmitteln, so spricht man von Vollkatalysatoren. Wird die Mischoxidaktivmasse auf vorgeformte inerte oder aktive Katalysatorträger aufgebracht und/oder darauf erzeugt, so spricht man von Schalenkatalysatoren. Schließlich seien noch die Trägerkatalysatoren erwähnt, bei denen die Mischoxidaktivmasse in die Poren inerter Trägerkörper aufgenommen und/oder in selbigen erzeugt wird. Die Formgebung bzw. das Aufbringen der Mischoxidmassen oder ihrer Vorläufer auf Trägerkörper ist allgemein bekannt und wird beispielsweise in der EP-A-714 700 und EP-A-17000 beschrieben.

Ausführliche Verfahrensbeschreibungen für die Herstellung von Katalysatorformkörpern erfindungsgemäß geeigneter Mischoxidaktivmassen finden sich z.B. in den Schriften EP-A 700 893, DE-A 10 063 162, DE-A 10 046 957, DE-A 19 948 523, EP-A 700 714, EP-A 417 723, DE-A 3300044; EP-A 552 287, EP-A 714 700, DE-A 10 059 713 und DE-A 10 051 419.

Die Katalysatorformkörper können entweder für sich oder im Gemisch mit inerten Formkörpern (z.B. den zur Herstellung von Schalenkatalysatoren verwendbaren inerten Trägerkörpern) zu Katalysatorfestbetten aufgeschüttet werden. Diese Katalysatorfestbetten können sich beispielsweise in den Rohren von Rohrbündelreaktoren (vgl. z.B. EP-A 700 893 und EP-A 700 714) oder auf den Horden von Hordenreaktoren befinden.

Als typische Geometrien für die Vollkatalysator-, Schalenkatalysator- und Trägerkatalysatorformkörper werden für Gasphasenpartialoxidationen Kugeln, Ringe und Zylinder empfohlen.

Die Größe der erfindungsgemäß zu verwendenden Katalysatorkörper ist in der Regel so bemessen, dass die Längstausdehnung (längste Verbindungslinie zweier auf der Oberfläche des Katalysatorkörpers befindlicher Punkte) 2 bis 12 mm, häufig 4 bis 8 mm beträgt.

Für das Katalysatorteilbett A sind als Katalysatoraktivmasse Mischoxide geeignet, die die Elemente Mo, Bi und Fe enthalten. Bevorzugt werden Mischoxide der allgemeinen Formel I

$$Mo_{12}Bi_aFe_bX^1_cX^2_dX^3_eX^4_fO_n \qquad (I),$$

in der die Variablen nachfolgende Bedeutung aufweisen:

$X^1$ =     Nickel und/oder Kobalt,

$X^2 =$    Thallium, ein Alkalimetall und/oder ein Erdalkalimetall,

$X^3 =$    Zink, Phosphor, Arsen, Bor, Antimon, Zinn, Cer, Blei und/oder Wolfram,

$X^4 =$    Silicium, Aluminium, Titan und/oder Zirkonium,

$a =$    0,5 bis 5,

$b =$    0,01 bis 5, vorzugsweise 2 bis 4,

$c =$    0 bis 10, vorzugsweise 3 bis 10,

$d =$    0 bis 2, vorzugsweise 0,02 bis 2,

$e =$    0 bis 8, vorzugsweise 0 bis 5,

$f =$    0 bis 10 und

$n =$    eine Zahl, die durch die Wertigkeit und Häufigkeit der von Sauerstoff verschiedenen Elemente in I bestimmt wird.

Sie sind in an sich bekannter Weise erhältlich (siehe z.B. die DE-A 4023239, EP-A-1005908 und EP-A-575897) und können z.B. entweder in Substanz zu Ringen oder Kugeln geformt oder in Gestalt von ring- oder kugelförmigen Schalenkatalysatoren, d.h., mit der Mischoxidaktivmasse beschichteten ring- oder kugelförmig vorgeformten, inerten Trägerkörpern, eingesetzt werden.

Prinzipiell können geeignete Mischoxidaktivmassen I in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter, reduzierender Atmosphäre (z.B. Gemisch aus Inertgas, $NH_3$, CO und/ oder $H_2$) erfolgen. Die Calcinationsdauer kann einige Minuten bis einige Stunden betragen und nimmt üblicherweise mit der Temperatur ab. Als Quellen für die elementaren Konstituenten der Mischoxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu vollständig gasförmig entweichenden Verbindungen zerfallen und/oder zersetzt werden können, können in das innige Trockengemisch zusätzlich eingearbeitet werden).

Günstige für die in Teilbett A zu verwendenden Katalysatoraktivmassen sind ferner Mischoxide der allgemeinen Formel III

$$[Bi_{a''}(Wolfram)_{b''}O_{x''}]_{p''} \, [(Molybdän)_{12}Z^3_{c''}Z^4_{d''}Fe_{e''}Z^5_{p''}Z^6_{g''}Z^7_{h''}O_{y''}]_{q''} \qquad (III),$$

in der die Variablen folgende Bedeutung haben:

$Z^3 =$    Nickel und/oder Kobalt,

$Z^4 =$    Thallium, ein Alkalimetall und/oder ein Erdalkalimetall,

$Z^5 =$    Phosphor, Arsen, Bor, Antimon, Zinn, Cer und/oder Blei,

$Z^6 =$    Silicium, Aluminium, Titan und/oder Zirkonium,

$Z^7 =$    Kupfer, Silber und/oder Gold,

$a'' =$    0,1 bis 1,

$b'' =$    0,2 bis 2,

$c'' =$    3 bis 10,

$d'' =$    0,02 bis 2,

$e'' =$    0,01 bis 5, vorzugsweise 0,1 bis 3,

$f'' =$    0 bis 5,

$g'' =$    0 bis 10,

$h'' =$    0 bis 1,

$x'',y''=$    zahlen, die durch die Wertigkeit und Häufigkeit der von Sauerstoff verschiedenen Element in III bestimmt werden, und

$p'',q''=$    Zahlen, deren Verhältnis $p''/q''$ 0,1 bis 5, vorzugsweise 0,5 bis 2 beträgt.

Erfinderungsgemäß besteht die Katalysatoraktivmasse des Teilbetts A ausschließlich aus Mischoxiden, die die Elemente Mo, Bi und Fe enthalten. Besonders bevorzugt besteht die Katalysatoraktivmasse ausschließlich aus Mischoxiden der allgemeinen Formeln I oder insbesondere III.

Für Katalysatorteilbett B sind als Katalysatoraktivmasse Mischoxide geeignet, die die Elemente Mo und V enthalten. Bevorzugt werden Mischoxide der allgemeinen Formel IV

$$Mo_{12}V_aX^1{}_bX^2{}_cX^3{}_dX^4{}_eX^5{}_fX^6{}_gO_n \qquad (IV),$$

in der die Variablen folgende Bedeutung haben:

$X^1 =$    W, Nb, Ta, Cr und/oder Ce,
$X^2 =$    Cu, Ni, Co, Fe, Mn und/oder Zn,
$X^3 =$    Sb und/oder Bi,
$X^4 =$    eines oder mehrere Alkalimetalle,
$X^5 =$    eines oder mehrere Erdalkalimetalle,
$X^6 =$    Si, A1, Ti und/oder Zr,
$a =$    1 bis 6,
$b =$    0,2 bis 4,
$c =$    0,5 bis 18,
$d =$    0 bis 40,
$e =$    0 bis 2,
$f =$    0 bis 4,
$g =$    0 bis 40 und
$n =$    eine Zahl, die durch die Wertigkeit und Häufigkeit der von Sauerstoff verschiedenen Elemente in IV bestimmt wird.

Bevorzugte Ausführungsformen innerhalb der Mischoxidaktivmassen IV sind jene, die von nachfolgenden Bedeutungen der Variablen der allgemeinen Formel IV erfaßt werden:

$X^1 =$    W, Nb, und/oder Cr,
$X^2 =$    Cu, Ni, Co, und/oder Fe,
$X^3 =$    Sb,
$X^4 =$    Na und/oder K,
$X^5 =$    Ca, Sr und/oder Ba,
$X^6 =$    Si, Al, und/oder Ti,
$a =$    1,5 bis 5,
$b =$    0,5 bis 2,
$c =$    0,5 bis 3,
$d =$    0 bis 2,
$e =$    0 bis 0,2,
$f =$    0 bis 1 und
$n =$    eine Zahl, die durch die Wertigkeit und Häufigkeit der von Sauerstoff verschiedenen Elemente in IV bestimmt wird.

Ganz besonders bevorzugte Mischoxidaktivmassen IV sind jedoch jene der allgemeinen Formel V

$$Mo_{12}V_{a'}Y^1{}_{b'}Y^2{}_{c'}Y^5{}_{f'}Y^6{}_{g'}O_{n'} \qquad (V)$$

mit

$Y^1 =$    W und/oder Nb,
$Y^2 =$    Cu und/oder Ni,
$Y^5 =$    Ca und/oder Sr,
$Y^6 =$    Si und/oder A1,
$a' =$    2 bis 4,
$b' =$    1 bis 1,5,
$c' =$    1 bis 3,
$f' =$    0 bis 0,5
$g' =$    0 bis 8 und
$n' =$    eine Zahl, die durch die Wertigkeit und Häufigkeit der von Sauerstoff verschiedenen Elementen in V bestimmt wird.

Die erfindungsgemäß geeigneten Mischoxidaktivmassen IV sind in sich bekannter, z.B. in der DE-A 4335973, EP-A-17000 oder in der EP-A 714700 offenbarter, Weise erhältlich.

Prinzipiell können erfindungsgemäß geeignete Mischoxidaktivmassen der allgemeinen Formel IV in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C unter Inertgas, oxidativen Atmosphäre wie z.B. Luft (Gemisch aus Inertgas und Sauerstoff) oder reduzierender Atmosphäre (z.B. Gemische aus Inertgas und reduzierenden Gasen wie $H_2$, $NH_3$, CO, Methan und/oder Acrolein oder die genannten reduzierend wirkenden Gase für sich) calciniert. Die Calcinationsdauer kann einige Minuten bis einige Stunden betragen und nimmt üblicherweise mit der Temperatur ab. Als Quellen für die elementaren Konstituenten der Mischoxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Günstige für die in Teilbett B zu verwendenden Katalysatoraktivmassen sind ferner Mischoxide der allgemeinen Formel VI,

$$[D]_p[E]_q \qquad (VI),$$

in der die Variablen folgende Bedeutung haben:

$D = Mo_{12}V_{a''}Z^1_{b''}Z^2_{c''}Z^3_{d''}Z^4_{e''}Z^5_{f''}Z^6_{g''}O_{x''}$ ,
$E = Z^7_{12}Cu_{h''}H_{i''}O_{y''}$ ,
$Z^1 = $ W, Nb, Ta, Cr und/oder Ce,
$Z^2 = $ Cu, Ni, Co, Fe, Mn und/oder Zn,
$Z^3 = $ Sb und/oder Bi,
$Z^4 = $ Li, Na, K, Rb, Cs und/oder H
$Z^5 = $ Mg, Ca, Sr und/oder Ba,
$Z^6 = $ Si, A1, Ti und/oder Zr,
$Z^7 = $ Mo, W, V, Nb und/oder Ta,

$a'' = $ 1 bis 8,
$b'' = $ 0,2 bis 5,
$c'' = $ 0 bis 23,
$d'' = $ 0 bis 50,
$e'' = $ 0 bis 2,
$f'' = $ 0 bis 5,
$g'' = $ 0 bis 50,
$h'' = $ 4 bis 30,
$i'' = $ 0 bis 20 und
$x'',y'' = $ Zahlen, die durch die Wertigkeit und Häufigkeit der von Sauerstoff verschiedenen Element in VI bestimmt werden und
$p,q = $ von Null verschiedene Zahlen, deren Verhältnis $p/q$ 160:1 bis 1:1 beträgt,

und die dadurch erhältlich sind, dass man eine Multimetalloxidmasse E

$$Z^7_{12}Cu_{h''}H_{i''}O_{y''} \qquad (E),$$

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a''}Z^1_{b''}Z^2_{c''}Z^3_{d''}Z^4_{e''}Z^5_{f''}Z^6_{g''} \qquad (D),$$

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Formung zur gewünschten Katalysatorgeometrie bei Temperaturen von 250 bis 600°C calciniert.

Bevorzugt sind Mischoxidaktivmassen VI, bei denen die Einarbeitung der vorgebildeten festen Ausgangsmasse 1 in eine wässrige Ausgangsmasse 2 bei einer Temperatur $\leq 70°C$ erfolgt. Eine detaillierte Beschreibung der Herstellung von Mischoxid VI-Aktivmassen enthalten z.B. die EP-A 668104, die DE-A 19736105 und die DE-A 19528646.

In einer bevorzugten Ausführungsform besteht die Katalysatoraktivmasse des Teilbetts B ausschließlich aus Mischoxiden der allgemeinen Formeln IV, V oder bevorzugt VI.

Vorzugsweise erfolgt die Durchführung des erfindungsgemäßen Verfahrens in mit den Katalysatoren beschichteten Rohrbündelreaktoren, wie sie z.B. die EP-A 700 714 und die EP-A 700 893 und die in diesen Schriften zitierte Literatur beschreiben.

Üblicherweise sind in den vorgenannten Rohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468 290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Abhängig von den verwendeten Mischoxiden werden die Partialoxidationen bei unterschiedlichen Temperaturen des Katalysatorteilbetts durchgeführt. Da es sich um eine exotherme Reaktion handelt, ist die Wärmeabführung wichtig. Durch die Reaktordimension oder die Verdünnung des Katalysators kann man die Temperatur des Wärmeaustauschmittels und die Heißpunkttemperatur beeinflussen.

Die Temperatur des Wärmeaustauschmittels ändert sich während des Kontakts mit den Kontaktrohren des Rohrbündels. Über ihre Eingangstemperatur (Erstkontakt) und die abgeführte Energiemenge, die durch die Durchflußmenge und Wärmekapazität des Wärmeaustauschmittels bedingt wird, kann man den Propenumsatz bzw. in Teilbett B den Acroleinumsatz steuern. Im folgenden soll unter der Temperatur des Wärmeaustauschmittels die maximale Temperatur des Wärmeaustauschmittels über den gesamten Kühlkreislauf verstanden werden.

Die Heißpunkttemperatur gibt den höchsten in den Kontaktrohren ermittelten Heißpunktwert wider. Zu ihrer Ermittlung wählt man beispielsweise 5 Kontaktrohre eines Rohrbündels, die im Rohrbündel äquidistant von ganz außen nach ganz innen radial liegen, aus und ermittelt den höchsten Wert.

Bevorzugt wird ein Verfahren, bei dem bei einfachem Durchlauf durch Teilbett A der Propenumsatz $\geq$ 97 mol-%, insbesondere $\geq$ 97,5 mol-%, besonders bevorzugt $\geq$ 98 mol-% und ganz besonders bevorzugt $\geq$98,5 mol-% ist.

Der Propenumsatz (Up) ist in dieser Schrift wie folgt definiert:

$$U_p \ (mol\text{-}\%) = \frac{Molzahl \ umgesetztes \ Propen}{Molzahl \ eingesetztes \ Propen} \cdot 100$$

Der Propenumsatz wird ermittelt, indem man am Ausgang des Katalysatorfestbetts A eine Probe für eine gaschromatographische Analyse entnimmt.

Der Propenumsatz kann über die Temperatur des Katalysators des Teilbetts A oder über die Katalysatorbelastung und damit die Verweilzeit geregelt werden. Bevorzugt wählt man eine Katalysatorbelastung von mindestens 70 l Propen/l Katalysator•Stunde. Katalysatorbelastungen oberhalb 300 sind in der Regel nicht zu empfehlen, da die bei solch hohen Propenumsätzen auftretenden Temperaturen sich nachteilig auf die Acrylsäureausbeute auswirken und darüberhinaus die Lebensdauer des Katalysators beeinflussen können. Besonders bevorzugt wählt man bei Mischoxidkatalysatoren der allgemeinen Formel I bevorzugt II, insbesondere III Katalysatorbelastungen im Bereich von 80 bis 200 l/l•h.

Charakteristische Größen für die Katalysatortemperatur sind die Temperatur des Wärmeaustauschmittels und die Heißpunkttemperatur. Bevorzugt wählt man Temperaturen des Wärmeaustauschmittels $\geq$ 200°C, besonders bevorzugt im Bereich von 240 bis 330°C. Temperaturen oberhalb 350°C wirken sich in der Regel nachteilig auf die Lebensdauer des Katalysators aus. Bevorzugt wählt man diese Temperaturen des Wärmeaustauschmittels bei Mischoxidkatalysatoren der allgemeinen Formel I bevorzugt II, insbesondere III. Bevorzugt werden Heißpunkttemperaturen in Teilbett A von 350 bis 425°C, insbesondere von 360 bis 420°C. Der Druck mit dem das Eingangsgasgemisch eingeleitet wird, liegt im Bereich von 1 bis 4 bar und ist allgemein üblich. Vorzugsweise leitet man das Gasgemisch bei einem Druck von 1,8 bis 2,2 bar in den Reaktor. In einer bevorzugten Verfahrensvariante ist der Druck in beiden Teilbetten gleich. Es ist jedoch auch möglich, durch Wahl eines engeren oder breiteren Querschnitts den Druck in Teilbett B und damit die Verweilzeit zu ändern.

Es wurde ferner gefunden, dass man durch die Wahl der Reaktionsbedingungen in Teilbett B den Allylacrylatgehalt beeinflussen kann. So werden solche Verfahren bevorzugt, die zusätzlich in der 2. Oxidationsstufe einen hohen Acroleinumsatz haben. Es wurde gefunden, dass Verfahren mit einem Acroleinumsatz $\geq 97$ mol-% und einer Heißpunkt-temperatur in Teilbett B $\leq 315°C$ einen sehr geringen Nebenproduktgehalt an Allylacrylat haben.

Bevorzugt wird ein Acroleinumsatz nach einfachem Durchlauf durch Teilbett B von $\geq 97$ mol-%, bevorzugt $\geq$ 97,5 mol-%, besonders bevorzugt $\geq 98$ mol-%, insbesondere $\geq 98,5$ mol-%.

Der Acroleinumsatz ($U_A$) ist in dieser Schrift wie folgt definiert:

$$U_A \; (mol\text{-}\%) \; = \; \frac{\text{Molzahl umgesetztes Acrolein}}{\text{Molzahl eingesetztes Acrolein}} \cdot 100$$

Der Acroleinumsatz wird ermittelt, indem man am Ausgang des Katalysatorfestbetts B eine Probe für eine gaschromatographische Analyse entnimmt.

Bevorzugt werden Heißpunkttemperaturen im Bereich von 250 bis 315°C, insbesondere von 280 bis 310°C.

Die Eintrittstemperatur des Wärmeaustauschmittels in den Reaktor wird dabei in an sich bekannter Weise so gewählt, dass sich bei gegebener Katalysatorbeschickung und vorgegebener Propen-Belastung das zur Erzielung des geforderten Acrolein-Umsatzes erforderliche Reaktionstemperaturprofil einstellt. So ist beispielsweise eine Variante die Heißpunkttemperatur über die Energieabführung zu regeln. Bei gleicher Verweilzeit und damit auch gleicher Katalysatorbelastung wie in Stufe 1 wird dies bevorzugt bei Badtemperaturen des Wärmeaustauschmittels $\leq 250°C$ bevorzugt 245 - 250°C im gesamten Kühlkreislauf erzielt. In der Regel haben die beiden Teilbetten A und B voneinander getrennte Kühlkreisläufe.

Die Heißpunkttemperatur kann beispielsweise auch durch die Katalysatorbelastung oder die Verdünnung des Katalysators reguliert werden. Die geschieht in an sich bekannter Weise.

Besonders bevorzugt wird ein Acroleinumsatz nach einfachem Durchlauf durch Teilbett B von $\geq 97$ mol-% und einer maximalen Heißpunkttemperatur im Teilbett B $\leq 315°C$ für Mischoxidkatalysatoren der allgemeinen Formel IV, bevorzugt V insbesondere VI.

Das erfindungsgemäße Verfahren zeichnet sich durch einen geringen Gehalt an störenden Nebenprodukten aus. So erzielt man hiermit einen Propionsäuregehalt $\leq 400$ ppm und einen Allylacrylatgehalt $\leq 100$ ppm.

Beispiele 1 - 3

Ein Reaktionsrohr (V2A Stahl, 30 mm Außendurchmesser; 2 mm Wandstärke; 29 mm Innendurchmesser, Länge: 439 cm) wurde von unten nach oben auf einem Kontaktstuhl (44 cm Länge) zunächst auf einer Länge von 30 cm mit eine rauhe Oberfläche aufweisenden Steatitkugeln (4 bis 5 mm Durchmesser; Inertmaterial zum Erwärmen des Reaktionsgasausgangsgemisches) und anschließend auf einer Länge von 270 cm (dem Katalysatorfestbett A) mit den nach EP-A-0 575 897 Beispiel 1 hergestellten Katalysatorkugeln (Aktivmasse des Katalysators ist ein Mischoxid aus Bi, W, Mo, Fe, Co und K) beschickt, bevor die Beschickung auf einer Länge von 30 cm mit den vorgenannten Steatitkugeln als Nachschüttung abgeschlossen wurde. Die verbleibende Kontaktrohrlänge wurde leer belassen. Es folgte ein zweites Reaktionsrohr (V2A Stahl, 30 mm Außendurchmesser, Länge: 439 cm), das auf einer Länge von 300 cm (dem Katalysatorfestbett B) mit dem nach EP-A-0 017 000 hergestellten Katalysatorkugeln (Aktivmasse des Katalysators ist ein Mischoxid aus Mo, V, W und Cu) beschickt, bevor die Beschickung auf einer Länge von 30 cm mit den vorgenannten Steatitkugeln abgeschlossen wurde.

Die Teile der Reaktionsrohre, die mit Feststoff beschickt waren, wurden mit je einem Salzbad thermostatisiert. Die an Feststoff freien Enden des Reaktionsrohres wurden mit unter erhöhtem Druck befindlichem Wasserdampf auf 220°C gehalten.

Das vorstehend beschriebene Reaktionsrohr wurde mit einem Reaktionsgasausgangsgemisch der nachfolgenden Zusammensetzung kontinuierlich beschickt:

6,5 Vol.-% Propen,
3,1 Vol.-% $H_2O$,
0,5 Vol.-% CO,
1,2 Vol.-% $CO_2$,
0,04 Vol.-% Acrolein,
10,7 Vol.-% $O_2$ und
als Restmenge 100 Vol.-% molekularer Stickstoff.

Die Belastung des Katalysatorfestbetts wurde zu 100 N1 Propen/l•h gewählt. Dem Produktgasgemisch wurde am Ausgang des Katalysatorfestbetts A eine kleine Probe für eine gaschromatographische Analyse entnommen. Die Temperatur des Salzbades wurde so eingestellt, dass der Propenumsatz in allen Fällen bei einmaligem Durchgang den gewünschten Wert betrug. Die dazu erforderlichen Temperaturen sind der nachfolgenden Tabelle zu entnehmen.

Dem Produktgasgemisch wurde am Ausgang des Katalysatorfestbetts B eine kleine Probe für die gaschromatographische Analyse (Acroleinumsatz, Allylacrylat- und Propionsäuregehalt) entnommen.

Um eine Vergleichbarkeit der Versuche zu gewähren wurde die Temperatur des Katalysatorfestbetts B so gewählt, dass der Acroleinumsatz in allen Fällen im Bereich von 99,5 bis 99,7 mol-% lag.

Die Heißpunkttemperatur wurde mit Hilfe eines Thermoelements im Reaktorrohr bestimmt.

Die nachfolgende Tabelle 1 zeigt die Abhängigkeit des Allylacrylat- und Propionsäuregehalts vom Propenumsatz in mol-%.

Tabelle 1

| | Salzbadtemperatur | | Heißpunkttemperatur | | Propenumsatz | Allylacrylat | Propionsäure |
|---|---|---|---|---|---|---|---|
| | Stufe 1 | Stufe 2 | Stufe 1 | Stufe 2 | | | |
| Bsp. 1 | 302°C | 248°C | 384°C | 304°C | 97,9 % | 51 ppm | 207 ppm |
| Vergleichs-bsp. 2 | 298°C | 250°C | 373°C | 317°C | 96,1 % | 104 ppm | 429 ppm |
| Vergleichs-bsp. 3 | 296°C | 253°C | 366°C | 317°C | 92,1 % | 132 ppm | 623 ppm |

Die Selektivität S ist wie folgt definiert:

$$S \text{ (mol-\%)} = \frac{\text{Molzahl Propen umgesetzt zu Acrolein/Acrylsäure}}{\text{Molzahl Propen/Acrolein insgesamt umgesetzt}} \cdot 100$$

Die erfindungsgemäßen Verfahren zeichnen sich durch eine hohe Selektivität (> 90 mol-%) bei niedrigen Nebenproduktgehalten aus.

Beispiele 4 - 6

Analog den Beispiele 1 bis 3 werden die Temperaturen des Katalysatorfestbett A so gewählt, dass der Propenumsatz im Bereich von 97,7 bis 97,9 mol-% lag. Die nachfolgende Tabelle 2 zeigt die Abhängigkeit des Allylacrylatgehalts von Acrolein-Umsatz in mol-% bei Heißpunkttemperaturen von 302 bis 306°C.

Tabelle 2

| Bsp. | Propenumsatz | Salzbadtemperatur | | Heißpunkttemperatur Stufe 2 | Acroleinumsatz | Allylacrylat |
|------|-------------|-------------------|---------|------------------------------|----------------|--------------|
|      |             | Stufe 1 | Stufe 2 |                              |                |              |
| 4 | 97,7 % | 304°C | 250°C | 302°C | 99,6 % | 48 ppm |
| 5 | 97,9 % | 306°C | 247°C | 304°C | 98,6 % | 55 ppm |
| 6 | 97,8 % | 305°C | 246°C | 306°C | 97,9 % | 61 ppm |

**Patentansprüche**

1. Verfahren zur Herstellung von Acrylsäure aus Propen mittels Gasphasenoxidation, indem man ein Propen und Wasserdampf enthaltendes Ausgangsgasgemisch, dessen molares Propen/Wasserdampfverhältnis $\geq 1$ ist und dessen Propangehalt $\leq 5$ % ist, bei erhöhter Temperatur durch ein Katalysatorfestbett mit aufeinanderfolgenden Katalysatorteilbetten A und B, wobei das zuerst durchlaufende Teilbett A als Aktivmasse ein die Elemente Mo, Bi und Fe enthaltendes Mischoxid und das Teilbett B als Aktivmasse ein die Elemente Mo und V enthaltendes Mischoxid enthält, derart führt, dass bei einfachem Durchlauf durch Teilbett A mindestens 96,5 mol.-% Propen umgesetzt werden.

2. Verfahren nach Anspruch 1, **dadurch gekennzeichnet, dass** der Propen-Umsatz nach einfachem Durchlauf durch Teilbett A $\geq 97,0$ mol-% ist.

3. Verfahren nach Anspruch 1 oder 2, **dadurch gekennzeichnet, dass** das Propen/Wasserdampfverhältnis im Ausgangsgasgemisch $\geq 1,5$ ist.

4. Verfahren nach einem der Ansprüche 1 bis 3, **dadurch gekennzeichnet, dass** die Katalysatorbelastung 70 - 300 1 Propen/1 Katalysatorstunde beträgt.

5. Verfahren nach einem der Ansprüche 1 bis 4, **dadurch gekennzeichnet, dass** die Aktivmasse des Teilbetts A nur aus den die Elemente Mo, Bi und Fe enthaltenden Mischoxiden besteht.

6. Verfahren nach einem der Ansprüche 1 bis 5, **dadurch gekennzeichnet, dass** die Aktivmasse des Teilbetts B nur aus den die Elemente Mo und V enthaltenden Mischoxiden besteht.

7. Verfahren nach einem der Ansprüche 1 bis 6, **dadurch gekennzeichnet, dass** die Aktivmasse des Teilbett A ein Mischoxid der allgemeinen Formel I

$$Mo_{12}Bi_aFe_bX^1{}_cX^2{}_dX^3{}_eX^4{}_fO_n \qquad (I),$$

mit

$X^1$ = Nickel und/oder Kobalt,
$X^2$ = Thallium, ein Alkalimetall und/oder ein Erdalkalimetall,
$X^3$ = Zink, Phosphor, Arsen, Bor, Antimon, Zinn, Cer, Blei und/oder Wolfram,
$X^4$ = Silicium, Aluminium, Titan und/oder Zirkonium,
a = 0,5 bis 5,
b = 0,01 bis 5
c = 0 bis 10,
d = 0 bis 2,
e = 0 bis 8,
f = 0 bis 10 und
n = eine Zähl, die durch die Wertigkeit und Häufigkeit der von Sauerstoff verschiedenen Elemente in I bestimmt wird,

ist.

**8.** Verfahren nach einem der Ansprüche 1 bis 7, **dadurch gekennzeichnet, dass** die Aktivmasse des Teilbetts B ein Mischoxid der allgemeinen Formel IV

$$Mo_{12}V_aX^1_bX^2_cX^3_dX^4_eX^5_fX^6_gO_n \qquad (IV),$$

mit

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle,
$X^5$ = eines oder mehrere Erdalkalimetalle,
$X^6$ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 4 und
n = eine Zahl, die durch die Wertigkeit und Häufigkeit der von Sauerstoff verschiedenen Elemente in IV bestimmt wird,

ist.

**9.** Verfahren nach einem der Ansprüche 1 bis 8, **dadurch gekennzeichnet, dass** die Oxidation von Acrolein zu Acrylsäure in Teilbett B mit einem Acroleinumsatz $\geq$ 97 mol-% und einer Heißpunkttemperatur $\leq$ 315°C durchgeführt wird.

**Claims**

**1.** A process for the preparation of acrylic acid from propene by means of gas-phase oxidation, in which a starting gas mixture comprising propene and steam and whose molar propene/steam ratio is $\geq$ 1 and whose propane content is $\leq$ 5% is passed at elevated temperatures through a fixed catalyst bed comprising successive catalyst part-beds A and B, the part-bed A passed through first comprising, as active material, a mixed oxide comprising the elements Mo, Bi and Fe and the part-bed B comprising, as active material, a mixed oxide comprising the elements Mo and V, in such a way that at least 96.5 mol% of propene are converted in a single pass through part-bed A.

**2.** The process according to claim 1, wherein the propene conversion after a single pass through part-bed A is $\geq$ 97.0 mol%.

**3.** The process according to claim 1 or 2, wherein the propene/steam ratio in the starting mixture is $\geq$ 1.5.

4. The process according to any of claims 1 to 3, wherein the catalyst space velocity is 70 - 300 1 of propene per 1 of catalyst per hour.

5. The process according to any of claims 1 to 4, wherein the active material of part-bed A consists only of the mixed oxides comprising the elements Mo, Bi and Fe.

6. The process according to any of claims 1 to 5, wherein the active material of part-bed B consists only of the mixed oxides comprising the elements Mo and V.

7. The process according to any of claims 1 to 6, wherein the active material of part-bed A is a mixed oxide of the formula I

$$Mo_{12}Bi_aFe_bX^1_cX^2_dX^3_eX^4_fO_n \qquad (I),$$

where

$X^1$ is nickel and/or cobalt,
$X^2$ is thallium, an alkali metal or an alkaline earth metal,
$X^3$ is zinc, phosphorus, arsenic, boron, antimony, tin, cerium, lead and/or tungsten,
$X^4$ is silicon, aluminum, titanium and/or zirconium,
a is from 0.5 to 5,
b is from 0.01 to 5,
c is from 0 to 10,
d is from 0 to 2,
e is from 0 to 8,
f is from 0 to 10 and
n is a number which is determined by the valency and
frequency of the elements other than oxygen in I.

8. The process according to any of claims 1 to 7, wherein the active material of the part-bed B is a mixed oxide of the formula IV

$$Mo_{12}V_aX^1_bX^2_cX^3_dX^4_eX^5_fX^6_gO_n \qquad (IV),$$

where

$X^1$ is W, Nb, Ta, Cr and/or Ce,
$X^2$ is Cu, Ni, Co, Fe, Mn and/or Zn,
$X^3$ is Sb and/or Bi,
$X^4$ is one or more alkali metals,
$x^5$ is one or more alkaline earth metals,
$X^6$ is Si, Al, Ti and/or Zr,
a is from 1 to 6,
b is from 0.2 to 4,
c is from 0.5 to 18,
d is from 0 to 40,
e is from 0 to 2,
f is from 0 to 4,
g is from 0 to 4 and
n is a number which is determined by the valency and
frequency of the elements other than oxygen in IV.

9. The process according to any of claims 1 to 8, wherein the oxidation of acrolein to acrylic acid in part-bed B is carried out with an acrolein conversion of $\geq 97$ mol% and a hotspot temperature of $\leq 315°C$.

**Revendications**

1. Procédé de préparation d'acide acrylique à partir de propène par oxydation en phase gazeuse, dans lequel on envoie un mélange gazeux de départ contenant du propène et de la vapeur d'eau, dont la proportion propène/ vapeur d'eau est ≥ 1 et dont la teneur en propane est ≤ 5 %, à température élevée, dans un lit fixe de catalyseur avec des lits partiels de catalyseur successifs A et B, où le lit partiel A parcouru en premier contient en tant que masse active un oxyde mixte contenant les éléments Mo, Bi et Fe et le lit partiel B contient en tant que masse active un oxyde mixte contenant l'un des éléments Mo et V, de manière à ce que pour un simple passage dans le lit partiel A, au moins 96,5 % molaires du propène soient convertis.

**2.** Procédé suivant la revendication 1, **caractérisé en ce que** la conversion du propène, après un simple passage dans le lit partiel A est ≥ 97,0 % molaires.

**3.** Procédé suivant la revendication 1 ou 2, **caractérisé en ce que** la proportion de propène/vapeur d'eau dans le mélange gazeux de départ est ≥ 1,5.

**4.** Procédé suivant l'une quelconque des revendications 1 à 3, **caractérisé en ce que** la charge du catalyseur est de 70 à 300 1 de propène/1 de catalyseur.

**5.** Procédé suivant l'une quelconque des revendications 1 à 4, **caractérisé en ce que** la masse active du lit partiel A consiste uniquement en oxydes mixtes contenant les éléments Mo, Bi et Fe.

**6.** Procédé suivant l'une quelconque des revendications 1 à 5, **caractérisé en ce que** la masse active du lit partiel B consiste uniquement en oxydes mixtes contenant les éléments Mo et V.

**7.** Procédé suivant l'une quelconque des revendications 1 à 6, **caractérisé en ce que** la masse active du lit partiel A est un oxyde mixte de la formule générale I

$$MO_{12}Bi_aFe_bX^1_cX^2_dX^3_eX^4_fO_n \qquad (I),$$

dans laquelle :

X$^1$ = nickel et/ou cobalt,
x$^2$ = thallium, métal alcalin et/ou métal alcalinoterreux,
X$^3$ = zinc, phosphore, arsenic, bore, antimoine, étain, cérium, plomb et/ou tungstène,
X$^4$ = silicium, aluminium, titane et/ou zirconium,
a = 0,5 à 5,
b = 0,01 à 5,
c = 0 à 10,
d = 0 à 2,
e = 0 à 8,
f = 0 à 10 et
n = un nombre déterminé par la valence et la fréquence des éléments différents de l'oxygène dans I.

**8.** Procédé suivant l'une quelconque des revendications 1 à 7, **caractérisé en ce que** la masse active du lit partiel B est un oxyde mixte de la formule générale IV

$$Mo_{12}V_aX^1_bX^2_cX^3_dX^4_eX^5_fX^6_gO_n \qquad (IV)$$

dans laquelle :

X$^1$ = W, Nb, Ta, Cr et/ou Ce,
X$^2$ = Cu, Ni, Co, Fe, Mn et/ou Zn,
X$^3$ = Sb et/ou Bi,
X$^4$ =un ou plusieurs métaux alcalins,
X$^5$ = un ou plusieurs métaux alcalino-terreux,
X$^6$ = Si, Al, Ti et/ou Zr,
a = 1 à 6,
b = 0,2 à 4,
c = 0,5 à 18,
d = 0 à 40,

e = 0 à 2,

f = 0 à 4,

g = 0 à 4 et

n = un nombre déterminé par la valence et 1a fréquence des éléments différents de l'oxygène dans IV.

9. Procédé suivant l'une quelconque des revendications 1 à 8, **caractérisé en ce que** l'oxydation d'acroléine en acide acrylique dans le lit partiel B est entreprise avec une conversion d'acroléine ≥ 97 % molaires et une température de point chaud ≤ 315 °C.

**IN DER BESCHREIBUNG AUFGEFÜHRTE DOKUMENTE**

**In der Beschreibung aufgeführte Patentdokumente**

- JP 2000053611 A
- DE 2311883 A
- JP 2000169420 A
- US 4147885 A
- US 4031135 A
- EP 0253409 A
- DE 19955176 A
- EP 911313 A
- DE 1962431 A
- DE 2943707 A
- DE 1205502 A
- EP 0257565 A
- DE 2251364 A
- EP 0117146 A
- GB 1450986 B
- EP 0293224 A
- EP 714700 A
- EP 17000 A
- EP 700893 A
- DE 10063162 A
- DE 10046957 A
- DE 19948523 A
- EP 700714 A
- EP 417723 A
- DE 3300044 A
- EP 552287 A
- DE 10059713 A
- DE 10051419 A
- DE 4023239 A
- EP 1005908 A
- EP 575897 A
- DE 4335973 A
- EP 668104 A
- DE 19736105 A
- DE 19528646 A
- EP 468290 B
- EP 0575897 A
- EP 0017000 A